# EUROPEAN PATENT APPLICATION

(11) **EP 4 239 076 A1**
(43) Date of publication of application: **06.09.2023**
(21) Application number: 20959044.7
(22) Date of filing: 28.10.2020
(51) Int. Cl.: C12Q 1/00, C12M 1/34, G01N 33/531

(54) **MICROBEAD AND PREPARATION METHOD THEREFOR**

(71) Applicant: BGI Shenzhen, Shenzhen, Guangdong 518083 (CN)
(72) Inventor: WANG, Weimao, Shenzhen, Guangdong 518083 (CN); ZHANG, Wenwei, Shenzhen, Guangdong 518083 (CN)
(74) Representative: Zaboliene, Reda
(86) International application number: PCT/CN2020/124334
(87) International publication number: WO 2022/087881

(57) **Abstract**

A microbead used in biological detection is provided. The microbead includes a first coating layer (20), a magnetic layer (30), and a second coating layer (40). The first coating layer (20) includes a first top surface (21) and a first side surface (22) connecting the first top surface (21). The magnetic layer (30) is formed on the first top surface (21), and the magnetic layer (30) includes a second top surface (31) away from the first top surface (21) and a second side surface (32) connecting the second top surface (31). The second coating layer (40) includes a top wall (41) and a periphery (42). The top wall (41) is connected to the second top surface (31), and the periphery (42) is connected to the top wall (41), the second side surface (32), and the first side surface (22), so that the first coating layer (20) and the second coating layer (40) coat the magnetic layer (30) inside the microbead. A preparation method of the microbead is also provided. The utilization rate of the microbead is improved.

## Description

### FIELD

The subject matter relates to biological detection, and more particularly, to a microbead used in biological detection and a preparation method of the microbead.

### BACKGROUND

Microbeads (also known as microparticles) are functional carriers coated with bioactive groups, and can be dispersed in a base liquid to form a magnetic liquid material. The bioactive groups can be coupled with a variety of bioactive substances, and both have fluidity of liquid and characteristics of solid magnetic particle materials. Under the action of an external magnetic field, the microbeads can directionally move and aggregated. After removing the external magnetic field, the original structure and state can be restored, thus making the complex liquid-solid separation technology fast and simple. Targeted substances with high purity can be obtained by a simple elution process. At present, the microbeads have been widely used in fields such as immunoassay, nucleic acid separation and extraction, cell sorting, and enzyme immobilization.

In the prior art, the microbeads are generally prepared by a swelling method. That is, polymer microbeads (such as polystyrene microbeads) are used, which swell in a specific organic solvent environment to improve the permeability of the microbeads. Under this condition, Fe₃O₄ magnetic nanoparticles enter the microbeads. With the continuous volatilization of the organic solvent, the permeability of the microbeads returns to normal, and the magnetic nanoparticles are embedded therein. However, the diameter of the microbeads prepared by such method is too small, which affects a loading amount of a substance to be detected and thus results in a large consumption of the microbeads during each reaction.

On the other hand, multiplex detection is a very important means in immunological detection and molecular diagnostic detection. The multiplex detection refers to the simultaneous measurement of multiple analytes in a single detection. The multiplex detection uses multiple capture reagents. Each capture reagent has specificity for different target macromolecules. In the chip-based array multiple assays, each type of capture reagent is attached to a predetermined position of the chip. Multiple targets in the complex sample are quantified by measuring the signal of the detection molecule at each position of the corresponding type of capture reagent. In a suspension-array-based multiplex detection, the microparticles or microbeads are suspended in a solution required for the detection. Such microparticles or microbeads include identification elements (e.g., codes) that can be embedded, printed, or otherwise generated by one or more elements of the microparticles or microbeads. Each type of capture reagent is fixed to the microparticles having a same code, and the signal emitted by the detection molecules on the particles having a specific code can reflect the amount of the corresponding target.

At present, the existing microbead coding technology, such as Luminex technology, codes the microbeads by fluorescent staining. The ratio of fluorescent dyes is adjusted to obtain a variety of microbeads with different fluorescence spectra. The antibody molecules or gene probes for different substances to be detected are combined to specific coded microbeads through covalent cross-linking. Each coded microbead corresponds to a detection item, so as to achieve the purpose of quantitative detection. However, such fluorescence coding technology has the following shortcomings. 1) It is unstable and needs to be operated in a dark environment during the whole process. 2) It needs to cooperate with a flowcytometry fluorescence detection instrument, which has high price and high maintenance cost.

### SUMMARY

To overcome at least a portion of the above shortcomings, a preparation method of a microbead is needed.

In addition, a microbead obtained by the above preparation method is also needed.

The present disclosure provides a microbead, which includes a first coating layer, a magnetic layer, and a second coating layer. The first coating layer includes a first top surface and a first side surface connected to the first top surface. The magnetic layer is formed on the first top surface. The magnetic layer includes a second top surface away from the first top surface and a second side surface connected to the second top surface. The second coating layer includes a top wall and a periphery. The top wall connected to the second top surface. The periphery is connected to each of the top wall, the second side surface, and the first side surface, thereby causing the first coating layer and the second coating layer to cooperatively coat the magnetic layer inside the microbead.

In one possible implementation, coding patterns are provided on the periphery.

In one possible implementation, the periphery includes an inner area and an edge area surrounding the inner area. The edge area includes corner portions and side portions besides the corner portions. At least one coding bit is provided on at least one of the side portions. Each of the at least one coding bit is configured to engrave the coding patterns.

In one possible implementation, the magnetic layer includes a chromium layer, a first tantalum layer, an iron-nickel alloy layer, and a second tantalum layer successively stacked on the first coating layer.

The present disclosure further provides a preparation method of a microbead, which includes providing a semiconductor substrate, forming a plurality of first coating layers which are spaced from each other on the semiconductor substrate, wherein each of the first coating layers includes a first top surface far away from the semiconductor substrate and a first side surface connected to the first top surface. A magnetic layer is formed on the first top surface of each of the first coating layers, wherein the magnetic layer includes a second top surface far from the first top surface and a second side surface connected to the second top surface. A plurality of second coating layers is formed on the semiconductor substrate, wherein each of the second coating layers includes a top wall and a periphery, the top wall is connected to the second top surface, the periphery is connected to each of the top wall, the second side surface, and the first side surface; each of the first coating layers, the respective one of the magnetic layer, and the respective one of the second coating layers cooperatively form one microbead. Each microbead is separated from the semiconductor substrate.

In one possible implementation, a preparation method of the second coating layers includes forming a photoresist on the semiconductor substrate. A mask with the coding patterns is covered on the photoresist, the photoresist is etched by a photolithographic method, and the photoresist is etched to obtain the second coating layers, wherein the photolithographic method is configured to transfer the coding patterns of the mask to the periphery.

In one possible implementation, the periphery includes an inner area and an edge area surrounding the inner area. The edge area includes corner portions and side portions besides the corner portions. At least one coding bit is provided on at least one of the side portions. Each of the at least one coding bit is configured to engrave the coding patterns.

In one possible implementation, before forming the first coating layers, the preparation method further includes forming a sacrificial layer on the semiconductor substrate, wherein the first coating layers is formed on the sacrificial layer. After forming the second coating layers, the preparation method further includes placing the semiconductor substrate having the sacrificial layer and the microbeads in an etching solution, wherein the etching solution has an etching property selectivity for the sacrificial layer, and is configured to separate each microbead from the semiconductor substrate.

In one possible implementation, forming the first coating layers includes forming a photoresist on the sacrificial layer. The photoresist is etched by an exposure and development process to obtain the first coating layers.

In one possible implementation, forming the magnetic layer includes forming a magnetic material on the sacrificial layer, wherein the magnetic material covers the first top surface and the first side surface of each of the first coating layers. A protective layer is formed on the magnetic material, wherein an orthogonal projection of the protective layer onto the first coating layers is located in the first coating layers. A portion of the magnetic material not covered by the protective layer is removed, wherein a remaining portion of the magnetic material forms the magnetic layer. The protective layer is removed.

In one possible implementation, the magnetic material is formed by vacuum electroplating or magnetron sputtering.

In one possible implementation, the magnetic layer includes a chromium layer, a first tantalum layer, an iron-nickel alloy layer, and a second tantalum layer successively stacked on the first coating layers.

In the present application, the magnetic layer is embedded inside the microbead. The magnetic layer is used to make the microbead magnetic, so that the microbead can be absorbed to a magnet on the magnetic grate. Thus, the substance to be detected is separated from the reaction solution. The microbead prepared in the present application is in a shape that deviates from circle. Compared with a circular microbead, the ratio of the overall surface area of the non-circular bead with respect to the periphery of the microbead before encoding is smaller, and more coding bits can be provided on a same consumable area, thereby improving the utilization rate of the microbead. Furthermore, the present application uses photolithography to encode the coding patterns on the second coating layer, thereby achieving the multiplex detection of the substances to be detected. Also, the instability of the existing fluorescence coding technology is avoided, and the use of an expensive flowcytometry fluorescence detection instruments is also avoided, thereby reducing the costs.

### BRIEF DESCRIPTION OF THE DRAWINGS

FIG. 1 is a diagrammatic view of a semiconductor substrate according to an embodiment of the present application.
FIG. 2 is a diagrammatic view showing a sacrificial layer formed on the semiconductor substrate of FIG. 1.
FIG. 3 is a diagrammatic view showing a first coating layer formed on the sacrificial layer of FIG. 2.
FIG. 4 is a diagrammatic view showing a magnetic material formed on the sacrificial layer having the first coating layer of FIG. 3.
FIG. 5 is a diagrammatic view showing a protective layer formed on the magnetic material of FIG. 4.
FIG. 6 is a diagrammatic view showing the magnetic material not covered by the protective layer of FIG. 5 removed.
FIG. 7 is a diagrammatic view showing the protective layer of FIG. 5 removed.
FIG. 8 is a top view of FIG. 7.
FIG. 9 is a diagrammatic view showing a second coating layer formed on the sacrificial layer having the first coating layer and the magnetic layer of FIG. 7.
FIG. 10 is a top view of FIG. 9.
FIG. 11 is a top view of a microbead that separates from the semiconductor substrate of FIG. 10.
FIG. 12 is the top view of the microbead of FIG. 11.

Symbol description of main components:
microbead 1
semiconductor substrate 10
sacrificial layer 11
first coating layer 20
first top surface 21
first side surface 22
magnetic layer 30
second top surface 31
second side surface 32
second coating layer 40
top wall 41
periphery 42
coding pattern 420
magnetic material 310
protective layer 320
inner area 421
edge area 422
corner portion 4221
side portion 4222

Implementations of the application will now be described, by way of embodiments only, with reference to the drawings.

### DETAILED DESCRIPTION

Implementations of the disclosure will now be described, by way of embodiments only, with reference to the drawings. The described embodiments are only some embodiments of the present disclosure, rather than all the embodiments. The disclosure is illustrative only, and changes may be made in the detail within the principles of the present disclosure. It will, therefore, be appreciated that the embodiments may be modified within the scope of the claims.

It should be noted that when a component is referred to as being "formed on" another component, the component can be directly on another component or a middle component may exist therebetween.

An embodiment of the present application provides a preparation method of a microbead. According to different requirements, the sequence of different steps of the preparation method can be changed, and some steps can be omitted or combined with each other. The preparation method includes the following steps.

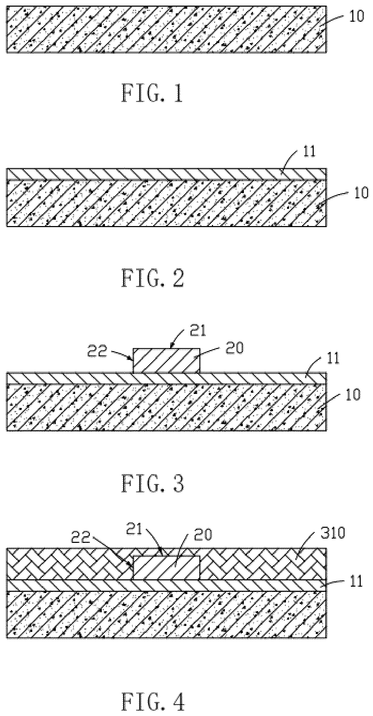

Step S1, referring to FIGS. 1 and 2, a semiconductor substrate 10 is provided, and a sacrificial layer 11 is formed on the semiconductor substrate 10.

In an embodiment, the semiconductor substrate 10 can be a silicon substrate, a gallium nitride substrate, a sapphire substrate, a ruby substrate, a quartz substrate, a gallium arsenide substrate, a silicon carbide substrate, a germanium substrate, or a diamond substrate. Optionally, the semiconductor substrate 10 is a silicon substrate.

In an embodiment, the sacrificial layer 11 is made of aluminum or zinc oxide, and has a thickness of 100 nm to 500 nm, preferably 300 nm to 400 nm. The sacrificial layer 11 can be formed by vacuum electroplating or magnetron sputtering. Optionally, the sacrificial layer 11 is made of aluminum.

Step S2, referring to FIG. 3, a number of first coating layers 20 spaced from each other are formed on the sacrificial layer 11 (FIG. 3 shows only one first coating layer 20). Each first coating layer 20 includes a first top surface 21 away from the sacrificial layer 11 and a first side surface 22 connected to the first top surface 21.

In an embodiment, the first coating layers 20 are formed on the sacrificial layer 11 in an array. A cross-section of each first coating layer 20 along an extension direction of the semiconductor substrate 10 is rectangular. The length and width of each first coating layer 20 is 10 microns × 5 microns. Each of the length and the width is defined as a dimension of the first coating layer 20 along the extension direction of the semiconductor substrate 10.

In an embodiment, the first coating layer 20 can be prepared as follows.

First, a photoresist is formed on the sacrificial layer 11. Specifically, the photoresist can be dripped onto the semiconductor substrate 10 that is driven to be rotated in a high speed through a spin coater (not shown). The spin coater uses centrifugal force to evenly coat the photoresist onto the sacrificial layer 11, so that the thickness of the photoresist is uniform. More specifically, the photoresist can be an ultraviolet photonegative photoresist, such as epoxy SU-8 resin, which is sensitive to electric beam, near-ultraviolet light, and ultraviolet light of 350-400 nm. In an embodiment, the thickness of the photoresist is 0.5 micron to 5 microns.

Then, the photoresist is etched by an exposure and development process to obtain the first coating layers 20 spaced from each other. Specifically, the semiconductor substrate 10 having the sacrificial layer 11 and the photoresist is placed in an exposure machine (not shown). A first mask (not shown) with a number of graphic openings covers on the photoresist, and the position of the graphic openings can correspond to the position where the first coating layers 20 need to be formed. The ultraviolet light is emitted towards the first mask, so that a portion of the photoresist exposed from the graphic openings of the first mask undergoes a cross-linking reaction and is then solidified under the ultraviolet light, while the remaining photoresist not covered by the first mask is not cross-linked (i.e., not solidified). The photoresist is then developed. When the photoresist is ultraviolet photonegative photoresist, the unsolidified photoresist covered by the first mask is etched and removed by a developer. At this time, the remaining photoresist not removed by the developer forms the first coating layers 20. In an embodiment, the ultraviolet light used during the exposure process has a wavelength of 365 nm and a power density is 12 mw/cm².

Step S3, referring to FIGS. 4 to 8., a magnetic layer 30 is formed on the first top surface 21 of each first coating layer 20. The magnetic layer 30 includes a second top surface 31 away from the first top surface 21 and a second side surface 32 connected to the second top surface 31.

The magnetic layer 30 includes a magnetic material. In an embodiment, the magnetic layer 30 includes a chromium (Cr) layer, a first tantalum (Ta) layer, an iron-nickel alloy layer, and a second tantalum layer successively stacked on the first coating layer 20. Optionally, the thicknesses of the chromium layer, the first tantalum layer, the iron-nickel alloy layer, and the second tantalum layer are in a ratio of 100 Å: 100 Å: 200 Å: 50 Å. The amounts of nickel and iron in the iron-nickel alloy layer are in a ratio of 80:20.

In an embodiment, the magnetic layer 30 can be prepared as follows.

As shown in FIG. 4, a magnetic material 310 is formed on the sacrificial layer 11. The magnetic material 310 covers the first top surface 21 and the first side surface 22 of each first coating layer 20. The magnetic material 310 can be formed by vacuum electroplating or magnetron sputtering.

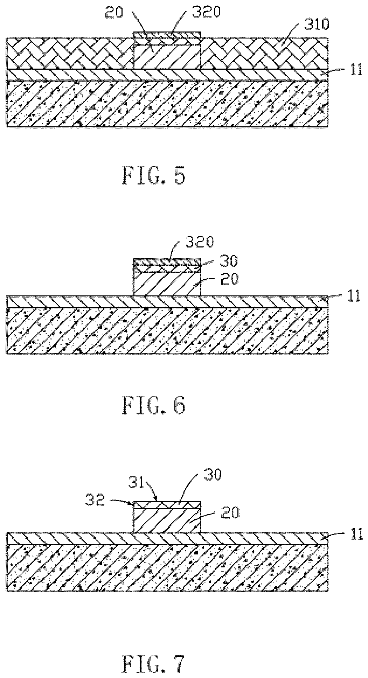

As shown in FIG. 5, a protective layer 320 is formed on the magnetic material 310. An orthogonal projection of the protective layer 320 onto the first coating layer 20 is located in the first coating layer 20. For example, the orthogonal projection of the protective layer 320 onto the first coating layer 20 may coincide with a portion of the first coating layer 20 or completely coincide with the first coating layer 20. In an embodiment, a protective material can be formed on the magnetic material 310 through a spin coater (not shown). Then, the protective material not corresponding to the first coating layer 20 can be etched by the exposure development method, and the remaining protective material forms the protective layer 320. More specifically, the developer used during the development process includes isopropanol (IPA) and methyl isobutyl ketone (MIBK), and the amounts of the isopropanol and the methyl isobutyl ketone are in a ratio of 3:1. The protective layer 320 can be made of polymethylmethacrylate (PMMA), and has a thickness of 100 nm to 1 micron, preferably 500 nm.

As shown in FIG. 6, the magnetic material 310 not covered by the protective layer 320 is removed, and the remaining magnetic material 310 forms the magnetic layer 30. When the orthogonal projection of the protective layer 320 onto the first coating layer 20 coincides with a portion of the first coating layer 20 (that is, another portion of the first coating layer 20 is not covered by the protective layer 320), the first coating layer 20 not covered by the protective layer 320 can also be further removed. In an embodiment, the magnetic material 310 and the first coating layer 20 not covered by the protective layer 320 can be removed by dry etching.

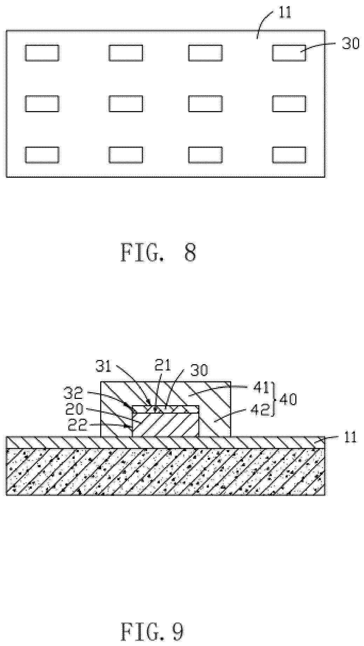

As shown in FIGS. 7 and 8, the protective layer 320 is removed. In an embodiment, at least the protective layer 320 is immersed in acetone and ultrasonically cleaned at 50 degrees Celsius to remove the protective layer 320.

Step S4, referring to FIG. 9, a number of second coating layers 40 are formed on the sacrificial layer 11 (FIG. 9 shows only one second coating layer 40). Each second coating layer 40 includes a top wall 41 and a periphery 42. The top wall 41 of each second coating layer 40 is connected to the second top surface 31 of one magnetic layers 30, and the periphery 42 of each second coating layer 40 is connected to the top wall 41, the second side surface 32 of the magnetic layer 30, and the first side surface 22 of the corresponding first coating layer 20.

In an embodiment, the second coating layer 40 also includes a photoresist that can be formed by the spin coater (not shown), and has a thickness of about 3 microns.

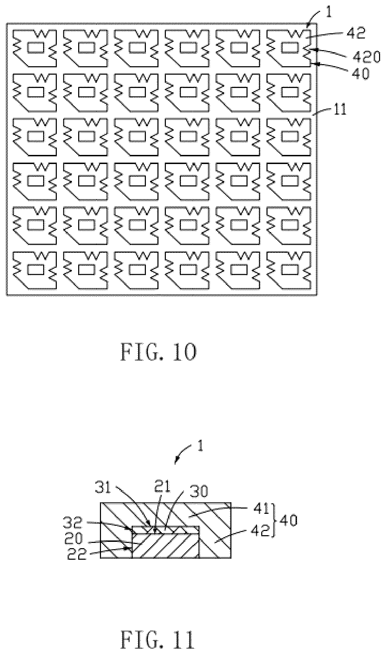

Furthermore, referring to FIG. 10, a second mask (not shown) with coding patterns covers on the photoresist. The photoresist is etched by photolithography and then solidified to obtain the second coating layers 40. The photolithography is used to transfer the coding patterns of the second mask to the periphery 42 of each second coating layer 40. At this time, each first coating layer 20, the corresponding magnetic layer 30, and the corresponding second coating layer 40 cooperatively form a microbead 1.

In an embodiment, a temperature for solidifying the second coating layers 40 is 165 degrees Celsius, and a period for solidifying the second coating layers 40 is 20 minutes.

The shape and size of each second coating layer 40 are set according to the shape and size of the microbead 1. Before coding, the cross-section of the second coating layer 40 along the extension direction of the semiconductor substrate 10 is non-circular. The non-circular shape can be rectangle (including square and rectangle), trapezoid, ellipse, or other regular or irregular shapes. In an embodiment, the cross-section of the second coating layer 40 along the extension direction of the semiconductor substrate 10 is rectangular. Therefore, the obtained microbead 1 is a non-circular microbead. Before coding, the length and width of the second coating layer 40 is 30 microns × 25 microns.

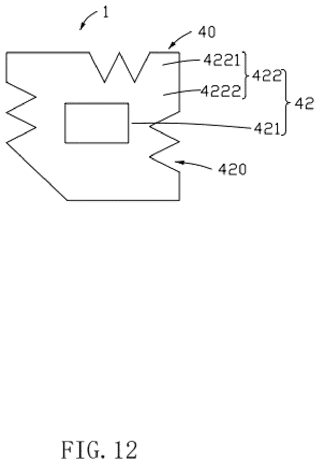

Furthermore, referring to FIG. 12, the periphery 42 of the second coating layer 40 of the microbead 1 includes an inner area 421 and an edge area 422 surrounding the inner area 421. The edge area 422 includes corner portions 4221 and side portions 4222 besides the corner portions 4221. The corner portions 4221 are where the four corners of the rectangle are located, and the side portions 4222 are where the long and short sides of the rectangle are located which connecting the corner portions 4221. At least one coding bit is provided on at least one side portions 4222, and each coding bit is used for engraving coding patterns 420. Each coding pattern 420 is a triangle with a vertex facing the inner area 421. In other embodiments, the coding pattern 420 can also be a triangle with a vertex far away from the inner area 421. The coding patterns 420 can also be replaced by rectangular patterns, trapezoidal patterns, or a combination thereof. It can be understood that by engraving the coding patterns 420 at different coding bits, the microbead 1 can obtain a variety of different codes.

Step S6, referring to FIG. 11, the semiconductor substrate 10 having the sacrificial layer 11 and the microbeads 1 is placed into an etching solution. The etching solution has an etching property selectivity for the sacrificial layer 11, which can separate the microbeads 1 from the semiconductor substrate 10.

The etching solution is used to etch the sacrificial layer 11 to separate each first coating layer 20 and the corresponding second coating layer 40 from the semiconductor substrate 10. At the same time, the etching solution will not etch the first coating layer 20, the second coating layer 40, or the semiconductor substrate 10. Specifically, the period for etching the sacrificial layer 11 in the etching solution is 30 minutes. After taking out the microbeads 1, an ultrasonic cleaning process is performed for 10 minutes. The microbeads 1 are then collected by centrifugation and placed on a magnetic grate for cleaning with deionized water.

In an embodiment, the etching solution includes a nitric acid solution, a phosphoric acid solution, hydrobromic acid solution, a mixed solution of phosphoric acid and acetic acid, a sodium hydroxide solution, an ammonium chloride solution, or an ammonia water. Optionally, the etching solution is a nitric acid solution having a concentration of 5%.

In another embodiment, the sacrificial layer 11 can also be omitted. At this time, the microbeads 1 can be separated from the semiconductor substrate 10 by other means (such as mechanical vibration).

Referring to FIGS. 11 and 12, a microbead 1 obtained by the above preparation method is also provided according to the present disclosure. The microbead 1 includes a first coating layer 20, a magnetic layer 30, and a second coating layer 40.

The first coating layer 20 includes a first top surface 21 and a first side surface 22 connected to the first top surface 21. The magnetic layer 30 is formed on the first side surface 22 of the first coating layer 20. The magnetic layer 30 includes a second top surface 31 away from the first top surface 21 and a second side surface 32 connected to the second top surface 31.

The second coating layer 40 includes a top wall 41 and a periphery 42. The top wall 41 is connected to the second top surface 31, and the periphery 42 is connected to the top wall 41, the second side surface 32, and the first side surface 22. As such, the first coating layer 20 and the second coating layer 40 cooperatively coat the magnetic layer 30 inside the microbead 1. Coding patterns 420 are provided on the periphery 42 of the second coating layer 40. The magnetic layer 30 is used to make the microbead 1 magnetic, so that the microbead 1 can be absorbed to a magnet on the magnetic grate. Thus, the substance to be detected is separated from the reaction solution.

The microbead 1 is non-circular, and the non-circular shape can be rectangular (including square, rectangle), trapezoidal, elliptical, and another regular or irregular shape.

In an embodiment, the microbead 1 is a rectangular microbead. Furthermore, the periphery 42 of the second coating layer 40 of the microbead 1 includes an inner area 421 and an edge area 422 surrounding the inner area 421. The edge area 422 includes corner portions 4221 and side portions 4222 besides the corner portions 4221. The corner portions 4221 are where the four corners of the rectangle are located. The side portions 4222 are where the long and short sides of the rectangle are located which connecting the corner portions 4221. A coding bit is provided on at least a side portion 4222, and each coding bit is used for engraving the coding patterns 420.

When in use, the active functional groups (such as carboxyl) is connected to the microbead 1 through a surface modification. The activator EDC/NHS is added. Then, the substances to be detected (such as antigen, antibody, nucleic acid molecule, etc.) is cross-linked with the microbead 1. The multiplex detection of the substances to be detected is realized by a double antibody sandwich method or a nucleic acid hybridization method.

In the present application, the magnetic layer 30 is embedded inside the microbead 1. The magnetic layer 30 is used to make the microbead 1 magnetic, so that the microbead 1 can be absorbed to a magnet on the magnetic grate. Thus, the substance to be detected is separated from the reaction solution. The microbead 1 prepared in the present application is in a shape that deviates from circle. Compared with a circular microbead, the ratio of the overall surface area of the non-circular bead 1 with respect to the periphery of the microbead 1 before encoding is smaller, and more coding bits can be provided on a same consumable area, thereby improving the utilization rate of the microbead 1. Furthermore, the present application uses photolithography to encode the coding patterns 420 on the second coating layer 40, thereby achieving the multiplex detection of the substances to be detected. Also, the instability of the existing fluorescence coding technology is avoided, and the use of an expensive flowcytometry fluorescence detection instruments is also avoided, thereby reducing the costs.

Even though information and advantages of the embodiments have been set forth in the foregoing description, together with details of the structures and functions of the embodiments, the disclosure is illustrative only. Changes may be made in detail, especially in matters of shape, size, and arrangement of parts within the principles of the present exemplary embodiments, to the full extent indicated by the plain meaning of the terms in which the appended claims are expressed.

## Claims

1. A microbead, **characterized in that**, comprises:
a first coating layer comprising a first top surface and a first side surface connected to the first top surface;
a magnetic layer formed on the first top surface, the magnetic layer comprising a second top surface away from the first top surface and a second side surface connected to the second top surface; and
a second coating layer comprising a top wall and a periphery, the top wall connected to the second top surface, the periphery connected to each of the top wall, the second side surface, and the first side surface, thereby causing the first coating layer and the second coating layer to cooperatively coat the magnetic layer inside the microbead.

2. The microbead according to claim 1, **characterized in that**, coding patterns are provided on the periphery.

3. The microbead according to claim 2, **characterized in that**, the periphery comprises an inner area and an edge area surrounding the inner area, the edge area comprises corner portions and side portions besides the corner portions, at least one coding bit is provided on at least one of the side portions, each of the at least one coding bit is configured to engrave the coding patterns.

4. The microbead according to claim 1, **characterized in that**, the magnetic layer comprises a chromium layer, a first tantalum layer, an iron-nickel alloy layer, and a second tantalum layer successively stacked on the first coating layer.

5. A preparation method of a microbead, **characterized in that**, comprises:
providing a semiconductor substrate, forming a plurality of first coating layers which are spaced from each other on the semiconductor substrate, wherein each of the first coating layers comprises a first top surface far away from the semiconductor substrate and a first side surface connected to the first top surface;
forming a magnetic layer on the first top surface of each of the first coating layers, wherein the magnetic layer comprises a second top surface far from the first top surface and a second side surface connected to the second top surface;
forming a plurality of second coating layers on the semiconductor substrate, wherein each of the second coating layers comprises a top wall and a periphery, the top wall is connected to the second top surface, the periphery is connected to each of the top wall, the second side surface, and the first side surface; each of the first coating layers, the respective one of the magnetic layer, and the respective one of the second coating layers cooperatively form one microbead;
separating each microbead from the semiconductor substrate.

6. The preparation method of the microbead according to claim 5, **characterized in that**, a preparation method of the second coating layers comprises:
forming a photoresist on the semiconductor substrate;
covering a mask with the coding patterns on the photoresist, etching the photoresist by a photolithographic method, and curing the photoresist to obtain the second coating layers, wherein the photolithographic method is configured to transfer the coding patterns of the mask to the periphery.

7. The preparation method of the microbead according to claim 6, **characterized in that**, the periphery comprises an inner area and an edge area surrounding the inner area, the edge area comprises corner portions and side portions besides the corner portions, at least one coding bit is provided on at least one of the side portions, each of the at least one coding bit is configured to engrave the coding patterns.

8. The preparation method of the microbead according to claim 5, **characterized in that**, before forming the first coating layers, the preparation method further comprises:
forming a sacrificial layer on the semiconductor substrate, wherein the first coating layers is formed on the sacrificial layer;
after forming the second coating layers, the preparation method further comprises:
placing the semiconductor substrate having the sacrificial layer and each microbead in an etching solution, wherein the etching solution has an etching property selectivity for the sacrificial layer, and is configured to separate each microbead from the semiconductor substrate.

9. The preparation method of the microbead according to claim 8, **characterized in that**, forming the first coating layers comprises:
forming a photoresist on the sacrificial layer;
etching the photoresist by an exposure and development process to obtain the first coating layers.

10. The preparation method of the microbead according to claim 8, **characterized in that**, forming the magnetic layer comprises:
forming a magnetic material on the sacrificial layer, wherein the magnetic material covers the first top surface and the first side surface of each of the first coating layers;
forming a protective layer on the magnetic material, wherein an orthogonal projection of the protective layer onto the first coating layers is located in the first coating layers;
removing a portion of the magnetic material not covered by the protective layer, wherein a remaining portion of the magnetic material forms the magnetic layer;
remove the protective layer.

11. The preparation method of the microbead according to claim 10, **characterized in that**, the magnetic material is formed by vacuum electroplating or magnetron sputtering.

12. The preparation method of the microbead according to claim 5, **characterized in that**, the magnetic layer comprises a chromium layer, a first tantalum layer, an iron-nickel alloy layer, and a second tantalum layer successively stacked on the first coating layers.
